(19) **Europäisches Patentamt / European Patent Office / Office européen des brevets**

(11) **EP 1 779 817 A1**

(12) **EUROPEAN PATENT APPLICATION**
published in accordance with Art. 158(3) EPC

(43) Date of publication:
**02.05.2007 Bulletin 2007/18**

(51) Int Cl.:
**A61F 2/84** *(2006.01)* **A61M 29/02** *(2006.01)*

(21) Application number: **05767392.3**

(22) Date of filing: **27.07.2005**

(86) International application number:
**PCT/JP2005/013754**

(87) International publication number:
**WO 2006/011523 (02.02.2006 Gazette 2006/05)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.07.2004 JP 2004222706**

(71) Applicant: **Kaneka Corporation
Kita-ku
Osaka-shi, Osaka 530-8288 (JP)**

(72) Inventor: **NAKANO, Ryoji
5660072 (JP)**

(74) Representative: **Gille Hrabal Struck Neidlein Prop Roos
Brucknerstrasse 20
40593 Düsseldorf (DE)**

(54) **STENT**

(57) It is intended to provide a stent to be used in a lumen which can uniformly cover an intralumen tissue, relieve troubles (for example, restenosis) in the intralumen tissue upon stent dilation, by easily compressed and fixed to a balloon or the like and safely move forward in the oral cavity of a patient's body, on which a sufficient amount of a drag can be fixed and from which the drug can be uniformly released into the intralumen tissue. Namely, a stent to be used in a lumen **characterized in that** it can be dilated from a first diameter in the compressed state to a second diameter in the enlarged state and, in the state of the first diameter, struts constituting the stent overlap together in the radius direction at least in one part.

Fig. 2

## EP 1 779 817 A1

**Description**

BACKGROUND OF THE INVENTION

Technical Field

The present invention relates to a stent for generally being transplanted to a living body.

Background Art

A stent is a medical device to treat various diseases resulting from stenosis or occlusion of blood vessels or other biological lumens by dilating the stenosed or occluded portion and to be retained at the place to maintain the lumen size, and there are various types, such as a coil-form stent having one piece of linear metal or polymer material, that fabricated by cutting and processed metal tube by laser, one formed by welding by laser and assembling linear members, that made by weaving a plurality of linear metal.

These can be classified into those dilated by balloon to which the stent is mounted (balloon expandable type) and those dilating by itself by removing members that suppress dilation from the outside (self-expandable type). The balloon expandable type is mounted to a balloon part of a thing with dilatable member like a balloon mounted at the vicinity of the head end of the intralumen catheter (balloon catheter), the catheter is allowed to advance to a treated portion in a patient body lumen, the balloon is dilated at the treated part, and in concert with this, the stent is dilated and indwelled. Then, the balloon is contracted and the catheter is removed. When the balloon is dilated, the dilation pressure is adjusted in accord with the state of intralumen tissue to be dilated and mechanical strength of the stent.

In recent years, these stents are popularly used for angioplasty particularly of the heart and carotid artery, and it has been indicated that stent placement can significantly reduce occurrence frequency of restenosis but it is the present condition that restenosis is brought about still now at a high probability. For example, to quote the cardiac coronary artery, even when stent placement is carried out, occurrence of restenosis is reported at a frequency of about 20 to 30%. This restenosis may be induced from biological blood vessel damage as well as blood vessel damage caused by stent placement . Typical blood stenosis/restenosis induced from blood vessel damage is assumed to be caused by internal smooth muscle cell proliferation. First of all, following blood vessel damage, smooth muscle cell proliferation begins; then, smooth muscle cells make the transition to a tunica intima. Then, smooth muscle cells in the tunica intima proliferate with substrate deposition and neointimal formation occurs. In addition, T-cell, macrophage, etc. are assumed to make the transition to the tunica intima, too.

In order to solve these problems, various stent designs (geometric shapes) have been proposed, and improved performance has been intended by a design which can manifest strength that does not yield to the intralumen tissue which is intended to be expanded by this, a design with flexibility that enables a stent to advance in a heavily curved intralumen tissue to a targeted region without any problem, a design that can uniformly cover the intralumen tissue, a design with less trouble to the intralumen tissue upon stent dilation. These can be disclosed, for example, in Patent Documents 1 through 9.

For example, Patent Document 10 disclosed an aim to coat a drug that restricts occlusion to a stent and to reduce the restenosis ratio. As drugs to restrict occlusion, anticoagulant, antiplatelet substance, anticonvulsant, antibacterial drug, antineoplastic, antimicrobe, anti-inflammatory agent, antimetabolic reaction, immunosuppressant, and other many drugs are under examination. To speak about immunosuppressant, attempts to coat stents with cyclosporin, tacrolimus (FK-506), sirolimus (rapamycin), mycophenolate mofetil , and their analogs and to reduce restenosis. Specifically, for example, Patent Document 11 disclosed a stent coated with sirolimus (rapamycin), which is known as an immunosuppressant, and Patent Document 12 disclosed a stent coated with taxol (paclitaxel) which is known as an antineoplastic. Furthermore, for example, Patent Documents 13 and 14 disclosed stents coated with tacrolimus (FK-506). However, these proposals include problems of a difficulty to apply an amount of drugs sufficient for treatment to stents, uniformly release drugs to the intralumen tissue, etc., and under the present circumstances, restenosis still occurs at a constant rate.

In addition, from a different viewpoint, stents are required for high holding force with a balloon or catheter. For example, in the case of a balloon expandable type, a stent is compressed and fixed to a balloon installed to the vicinity of the head end part of a catheter, and is allowed to advance to a treated region in the patient body lumen. In such event, the stent must be fixed to the balloon at a sufficient strength. In the event that the holding force between this balloon and the stent is insufficient, while the stent is allowed to advance in the patient body lumen, the stent generates deviation with respect to the balloon, and is unable to be dilated, and in the worst case, the stent may drop out from the balloon and possibly be released into the patient body lumen. Although thoroughgoing consideration is given to the holding force between this balloon and the stent, troubles such as deviation or dropout of the stent are still reported.

As mentioned so far, to have sufficient strength that does not yield to an intralumen tissue to be dilated, to have flexibility that allows a stent to advance in a heavily curved intralumen tissue and to advance to the targeted region without any trouble, to be able to uniformly cover the intralumen tissue, to be able to reduce damage to the intralumen tissue upon stent dilation, to be a stent that can apply as much amount of a drug as possible in a stent which is coated with a drug, to be able to uniformly release a drug into an intralumen tissue, and to have a holding force between this balloon and the stent that can safely advance a stent into a patient's body lumen have been the problems that must be solved in conventional technologies.

Furthermore, it has been reported that the stenosis ratio could be reduced as the stent strut thickness (thickness in the blood vessel radius direction) is reduced (for example, there is a report in non-Patent Document 1), and studies have been made on reducing the strut thickness, but needless to say, as the strut thickness is reduced, the stent strength is reduced, giving a problem of lowered visibility under X-ray illumination during operation. To avoid this, the stent width (blood vessel circumferential direction) must be increased, but this resulted in a problem in that a stent was unable to be compressed and fixed to a balloon in the event that the stent width was increased (a thick stent width causes a physical collision between struts when the stent is compressed and fixed to a balloon, which prevents the stent from being physically fixed). Consequently, in conventional technologies, the strut thickness was unable to be reduced more than a specified level.

Patent Document 1: Japanese Patent Application Laid-Open Publication No. H2-174859

Patent Document 2: Japanese Patent Application Laid-Open Publication No. H6-181993

Patent Document 3: Japanese Patent Application Laid-Open Publication No. H10-503676

Patent Document 4: Japanese Patent Application Laid-Open Publication No. H11-319112

Patent Document 5: Japanese Patent Application Laid-Open Publication No. H11-501551

Patent Document 6: Japanese Patent Application Laid-Open Publication No. 2001-224696

Patent Document 7: Japanese Patent Application Laid-Open Publication No. 2001-501494

Patent Document 8: Japanese Patent Application Laid-Open Publication No. 2001-501493

Patent Document 9: National Publication of Translated Version No. 2002-530146

Patent Document 10: National Publication of Translated Version No. H5-502179

Patent Document 11: Japanese Patent Application Laid-Open Publication No. H6-9390

Patent Document 12: National Publication of Translated Version No. H9-503488

Patent Document 13: International Publication No. WO02/065947 Patent Document 14: European Patent Publication No. 1254674 Non-Patent Document 1: Pache J. et al.: J Am Coll Cardiol 2003 Apr. 16; 41(8): 1289-92

DISCLOSURE OF INVENTION

Technical Problems to be Solved

What the present invention aims at solving in view of these conditions is to provide an intraluminal stent that has sufficient strength that does not yield to an intralumen tissue to be dilated, has flexibility that allows a stent to advance in a heavily curved intralumen tissue and to advance to the targeted region without any trouble, can uniformly cover the intralumen tissue, can reduce damage to the intralumen tissue upon stent dilation. Another problem to be solved by the present invention is to provide a stent that can apply as much amount of a drug as possible in a stent which is coated with a drug, can uniformly release a drug into an intralumen tissue. Another problem to be solved by the present invention is to provide an intraluminal stent that has a holding force between this balloon and the stent that can safely advance the stent into the patient's body lumen. Another problem to be solved by the present invention is to provide an intraluminal stent that is free of a problem of physical collision between struts, which prevents the stent from being physically fixed when the stent is compressed and fixed to a balloon, etc. even when the stent strut thickness is thin or the strut width is thick and at the same time that can compress and fix a stent to a balloon, etc. with sufficient strength.

Means to solve the problems

That is, the present invention (1) relates to an intraluminal stent, which is able to be dilated in the radius direction from a compressed first diameter to a dilated second diameter, and in the state of the first diameter, struts constituting the intraluminal stent overlap together in the radius direction at least in one part.

In addition, the present invention (2) relates to the intraluminal stent according to the invention (1), wherein the struts constituting the intraluminal stent do not overlap together in the radius direction in the state of the second diameter.

Furthermore, the present invention (3) relates to the intraluminal stent according to the invention (2), wherein the area of the portion with overlapping struts is larger than the area of the portion with no overlapping struts in the state of the first diameter.

Furthermore, the present invention (4) relates to the intraluminal stent according to the invention (1), wherein struts constituting the intraluminal stent are configured in nearly wave-like shapes.

In addition, the present invention (5) relates to the intraluminal stent according to the invention (1), wherein the intraluminal stent includes multiple cylindrical loop elements which can be independently dilated in a radius direction and the cylindrical loop elements are formed continuously nearly in the axial direction.

Furthermore, the present invention (6) relates to the intraluminal stent according to the invention (1), wherein the stent is formed by materials selected from stainless steel, nickel alloy, cobalt chromium alloy, and combinations of these.

Furthermore, the present invention (7) relates to the intraluminal stent according to the invention (1), wherein a drug that suppresses occlusion is fixed.

Furthermore, the present invention (8) relates to the intraluminal stent according to the invention (7), wherein the drug is fixed by biocompatible polymer.

Furthermore, the present invention (9) relates to the intraluminal stent according to the invention (7), wherein the drug is fixed by biodegradable polymer.

Furthermore, the present invention (10) relates to the intraluminal stent according to the invention (7), wherein the drug does not exist on the outer surface of the stent in the state of the first diameter but the drug exists on the outer surface of the stent in the state of the second diameter.

Effect of the invention

According to the present invention, a stent with thin strut thickness and with thick strut width can be provided by adopting a configuration in that struts of the compressed stent in the first diameter, struts constituting the intraluminal stent overlap together in the radius direction at least in one part. By this, the stent can uniformly cover the intralumen tissue, and can reduce damage, for example restenosis, to the intralumen tissue upon stent dilation. By adopting the relevant configuration, the physical collision between struts is difficult to occur, and the stent can be easily compressed and fixed to a balloon, and the stent can be allowed to advance safely in the patient body lumen. Furthermore, the stent has flexibility that enables the stent to advance in a heavily curved intralumen tissue without causing lowering of stent strength and X-ray visibility. Furthermore, the stent is able to have a sufficient amount of a drug fixed on the stent because the stent surface area is larger than conventional stents. Because the stent can increase the stent outer surface, too, when the stent is placed in the intralumen tissue, the stent can uniformly release the drug into the intralumen tissue more than before.

BRIEF DESCRIPTION OF THE DRAWINGS

Fig. 1 is a cross-sectional view in the axial direction in a contracted state of Comparison 1;
Fig. 2 is a development view in the contracted state of Comparison 1;
Fig. 3 is a cross-sectional view in the axial direction in a dilated state of Comparison 1;
Fig. 4 is a development view in the dilated state of Comparison 1;
Fig. 5 is a cross-sectional view in the axial direction in a contracted state of Example 1;
Fig. 6 is a development view in the contracted state of Example 1;
Fig. 7 is a cross-sectional view in the axial direction in a dilated state of Example 1;
Fig. 8 is a development view in the dilated state of Example 1;
Fig. 9 is a cross-sectional view in the axial direction in a contracted state of Example 2;
Fig. 10 is a development view in the contracted state of Example 2;
Fig. 11 is a cross-sectional view in the axial direction in a dilated state of Example 2;
Fig. 12 is a development view in the dilated state of Example 2;

Fig. 13 is a cross-sectional view in the axial direction in a contracted state of Example 3;

Fig. 14 is a development view in the contracted state of Example 3;

Fig. 15 is a cross-sectional view in the axial direction in a dilated state of Example 3;

Fig. 16 is a development view in the dilated state of Example 3;

Fig. 17 is a cross-sectional view in the axial direction in a contracted state of Example 4;

Fig. 18 is a development view in the contracted state of Example 4;

Fig. 19 is a cross-sectional view in the axial direction in a dilated state of Example 4;

Fig. 20 is a development view in the dilated state of Example 4;

Fig. 21 is a cross-sectional view in the axial direction in a contracted state of Example 5;

Fig. 22 is a development view in the contracted state of Example 5;

Fig. 23 is a cross-sectional view in the axial direction in a dilated state of Example 5;

Fig. 24 is a development view in the dilated state of Example 5;

Fig. 25 is a cross-sectional view in the axial direction in a contracted state of Example 6; and

Fig. 26 is a cross-sectional view in the axial direction in a dilated state of Example 6.

DESCRIPTION OF REFERENCE NUMERALS

| | |
|---|---|
| 101 | Catheter |
| 102 | Strut |
| 201 | Strut |
| 301 | Strut |
| 401 | Strut |
| 501 | Catheter |
| 502 | Strut |
| 601 | Strut |
| 701 | Strut |
| 801 | Strut |
| 901 | Catheter |
| 902 | Strut |
| 1001 | Strut |
| 1101 | Strut |
| 1201 | Strut |
| 1301 | Catheter |
| 1302 | Strut |
| 1401 | Strut |
| 1501 | Strut |
| 1601 | Strut |
| 1701 | Catheter |
| 1702 | Strut |
| 1801 | Strut |
| 1901 | Strut |
| 2001 | Strut |
| 2101 | Catheter |
| 2102 | Strut |
| 2201 | Strut |
| 2301 | Strut |
| 2401 | Strut |
| 2501 | Catheter |
| 2502 | Strut |
| 2503 | Drug layer |
| 2601 | Strut |
| 2602 | Drug layer |

BEST MODE FOR CARRYING OUT THE INVENTION

Hereinafter, embodiments of a stent related to the present invention will be described but the present invention shall not be restricted to these embodiments.

An embodiment of the present invention is an intraluminal stent to be transplanted in a body lumen, which can be dilated from a compressed first diameter to a dilated second diameter in the radius direction, and struts constituting the intraluminal stent overlap together in the radius direction at least in one part in the state of the first diameter. From the viewpoint to obtain a satisfactory effect of the present invention, it is preferable that struts constituting the intraluminal stent do not overlap together in the radius direction in the state of the second diameter. "Strut" is a term that indicates part constituting a stent, and a stent is configured by various struts (for example, bent struts, linear struts, wave-like struts, sine-wave-form struts, etc.).

In the mode of the present invention, the outer surface area of the stent practically increases when the stent is dilated from a compressed first diameter to a dilated second diameter. This is to deploy overlapping struts to the state in which struts do not overlap. By this, the stent struts is able to cover an intralumen tissue more uniformly and is able to reduce a degree of damage to the tissue. In addition, fixing a drug, for example, applying a drug, to the stent of the present invention enables the stent to release more uniformly the drug to the tissue.

There is no particular restriction to the compressed first diameter but from the viewpoint of clinical use, it can be set to not more than 1.2 mm, preferably to not more than 0.9 mm. The dilated second diameter should be chosen in accordance with the inner diameter of the patient's body lumen and completely differs in accord with lumens to be treated. For example, to take cardiac coronary artery as an example, the diameter is set to about 2.0 mm to 5.0 mm.

In working the present invention, the preferable strut ratio of width to thickness is 2 to 1 through 6 to 1 , and more preferably 3 to 1 through 5 to 1 , and in this range, the stent strength (rigidity to the external pressure) and flexibility are optimally balanced.

Possible examples of stent forming techniques include a laser processing method, electric discharge method, mechanical cutting method, etching method, etc. In addition, chamfering the strut end part in various polishing including electropolishing, etc. after a stent is formed is generally known by a person skilled in art, and it can be applied in the present invention.

The structural materials of a stent related to the present invention include unreactive polymers, or biocompatibility or non-compatibility metals or alloys. Examples of the polymer includes acrylonitrile polymers such as acrylonitrile butadiene styrene terpolymer, halogenated polymer, for example, polytetrafluoroethylene, polychrolotrifluoroethylene, tetrafluoroethylene copolymer, and hexafluoropropylene copolymer; polyamide; polysulfone; polycarbonate; polyethylene; polypropylene; polyvinylchloride-acryl copolymer; polycarbonate/acrylonitrile-butadiene-styrene; polystyrene, and the like. Examples of metal material useful for structural material include stainless steel, titanium, nickel, iridium, iridium-magnesium-oxide, niobium, platinum, tantalum, gold, and their alloys, and gold-plated iron alloys, platinum-plated iron alloys, cobalt chromium alloys, and titanium nitride coated stainless steel. Particularly preferable is a sterilization resistance material such as silicon-coated glass, polypropylene, vinyl chloride, polycarbonate, polysulphone, polymethylpenten, and the like. Preferably, the stent related to the present invention can be fabricated by stainless steel, Ni-Ti alloys and other nickel alloys, Cu-Al-Mn alloys, Co-Cr alloys, and other metals or combinations of these from the viewpoint of appropriate rigidity and elasticity, and for example, metals prescribed in JIS-G4303, ormetals, etc. prescribedinISO5832-5, ISO5832-6, and ISO5832-7 can be used.

For the stent shape (geometric shape) which is one of the embodiments of the present invention, a stent which includes multiple cylindrical loop elements which can be independently dilated in the radius direction and the cylindrical loop elements are formed continuously nearly in the axial direction can be mentioned.

The present invention can be fixed with a drug, for example, a drug that suppresses occlusion by application, etc. , and which can be selected from the following drug groups and combinations of these, for example, antiproliferative/ antimitotic agents that contain natural products, such as vinca alkaloid (that is, vinblastine, vincristine, vinorelbine, etc.), Paclitaxel, epipodophyllotoxin (that is, etoposide and teniposide), etc.; antibiotics such as dactinomycin (actinomycin D) , daunorubicin, doxorubicin and idarubicin, anthracycline, mitoxantrone, bleomycin, plicamycin (mithramycin), and mitomycin, etc.: enzymes (L-asparaginase that systematically metabolizes L-asparagine and which is not included in cells with no asparagines synthesis capability, etc.); antiproliferative/antimitotic alkylating agent such as nitrogen mustard (mechlorethamine,cyclophosphamide, and their analogs, melphalan, chlorambucil, etc.), ethyleneimine and methylmelamine (hexamethylmelamine and thiotepa, etc.), alkyl sulfonate busulfan, nitrosourea (carmustine (BCNU) and analogs , streptozocin, etc.), trazen-dacarbazine (DTIC), etc. (trazen, decarbazine); folic acid analogs (methotrexate, etc.), pyrimidine analogs (fluorouracil, floxuridine and cytarabine, etc.), purine analogs and related inhibitors (mercaptopurine, thioguanine, pentostatin and 2-chlorodeoxyadenosine {cladribine}), etc.; antimetabolite; platinum coordination complex (cisplatin, carboplatin, etc.), procarbazine, hydroxyurea, mitotane, aminoglutethimide; hormone (that is, estrogen, etc.); anticoagulant (heparin, synthetic heparin salt, and other thrombin inhibitor, etc.); fibrinogen degradation agent (tissue plasminogen activator, streptokinase and urokinase, etc.); antiplatelet agents (aspirin, dipyridamole, ticlopidine, clopidogrel, abciximab, etc.); migration suppressors; antisecretory agents (breveldin, etc.); anti-inflammatory agents such as corticosteroid (cortisol, cortisone, fludrocortisone, prednisone, prednisolone, $6\alpha$-methylprednisolone, triamcinolone, betamethasone and dexamethasone, etc.), nonsteroidal agents (salicylic acid derivative, that is aspirin, etc.); para-aminophenol derivatives, that is, acetaminophen; indole and indene acetate (indomethacin, sulindac, etodalac, etc.), heteroaryl acetate (tolmetin, diclofenac and ketorolac, etc.), arylpropionic acid (ibuprofen and derivatives, etc.), anthranilic acid (mefenamic acid and meclofenamic acid, etc.), enol acid (piroxicam, Tenoxicam, phenylbutazone, and oxyphenthatrazone, etc.), nabumetone, gold compounds (auranofin, (a-D-glucopyranosylthio) gold, sodium aurothiomalate, etc.); immunosuppressants (cyclosporin, tacrolimus (FK-506), sirolimus (rapamycin), azathioprine, mycophenolate mofetil, everolimus , ABT-578, CCI-779, AP23573, etc.); angiogenic agents: vascular endothelial growth factor (VEGF), fibroblast growth factor (FGF); nitrogen oxygen donor; antisense oligonucleotide, etc.

For methods for fixing drugs to stents, there are a method to physically fix drugs and a method to fix biocompatibility polymer and/or biodegradable polymer as a binder. For a coating method, a method to dip a stent in a solution or a method to atomize a solution to a stent by a sprayer is practicable.

For biocompatibility polymers used for the present invention, essentially, any biocompatibility polymers can be used as far as platelets are difficult to adhere, polymers do not display any irritating properties to tissues and can elute drugs, but examples of synthetic polymers include blends or block-copolymers of polyether type polyurethane and dimethylsilicon, polyurethane such as segmented polyurethane, etc., polyacrylic amide, polyethylene oxide, polyethylene carbonate, polypropylene carbonate, and other polycarbonates, and for natural biocompatible polymers, fibrin, gelatin, collagen, etc. can be used. These polymers can be used independently or in proper combinations. For biodegradable polymers used for the present invention, any biodegradable polymers can be used if polymers are decomposed enzymatically or nonenzymatically within an organism, decomposition products do not exhibit any toxicity, and polymers can release drugs. For example, any polymers which are properly selected from polylactic acid, polyglycolic acid, copolymers of polylactic acid and polyglycolic acid, collagen, gelatin, chitin, chitosan, hyaluronic acid, poly-L-glutamic acid, poly-L-lysine and other polyamino acid, starch, poly-ε-caprolactone, polyethylene succinate, poly-β-hydroxyalkanoate, etc. can be used. These polymers can be used independently or in proper combinations.

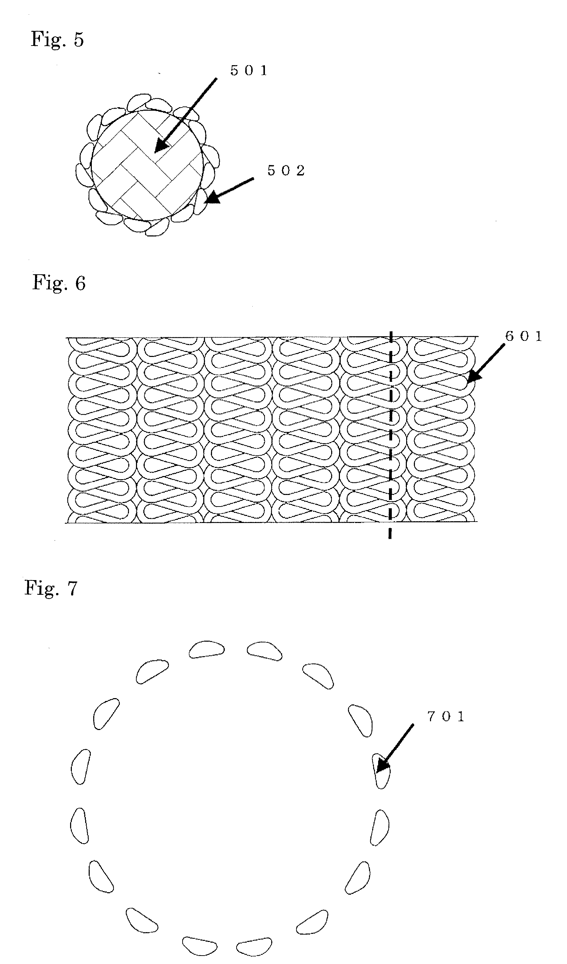

Figs. 5 through 8 show one embodiment of the present invention. For one embodiment of the present invention, struts constituting an intraluminal stent are formed by nearly wave shapes and a part of struts overlaps adjacent struts in the radius direction in the folded first radius. The cross-sectional shape of a strut may be square, oblong, triangular, trapezoid, and of shapes with those chamfered. In the dilated second radius, an intraluminal stent does not overlap in the radius direction and is dilated nearly uniformly. Preferably, the intraluminal stent of the present embodiment is fabricated by cobalt chromium alloy.

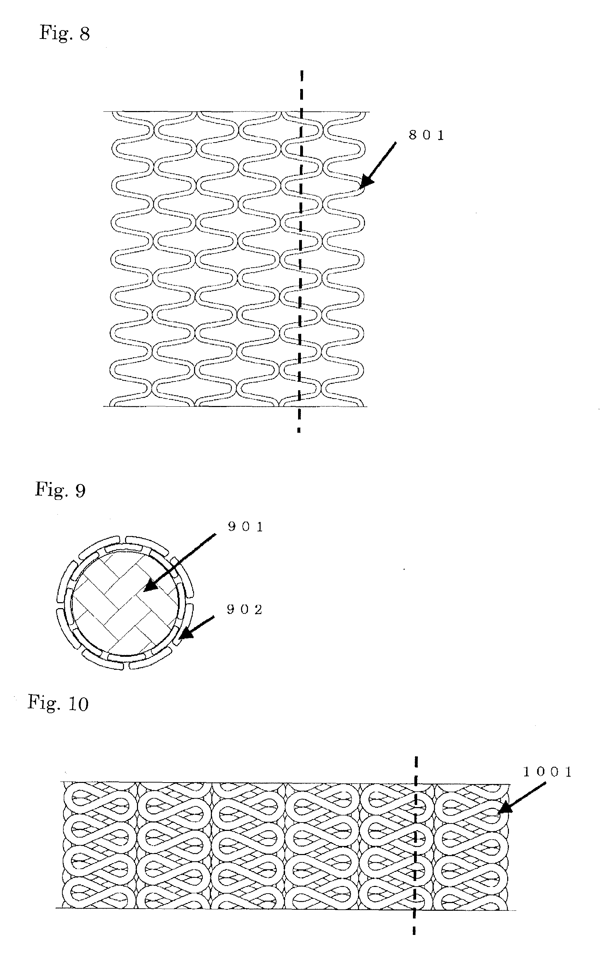

Figs. 9 through 12 show one embodiment of the present invention. For one embodiment of the present invention, struts constituting an intraluminal stent are formed by nearly wave shapes and adjacent struts overlap together in the radius direction in the folded first radius, and when a certain cross-section of the intraluminal stent is observed, struts are folded in two layers, outside and inside, in the radius direction. Furthermore, in the dilated second radius, an intraluminal stent does not overlap in the radius direction and is dilated nearly uniformly. Preferably, the intraluminal stent of the present embodiment is fabricated by cobalt chromium alloy.

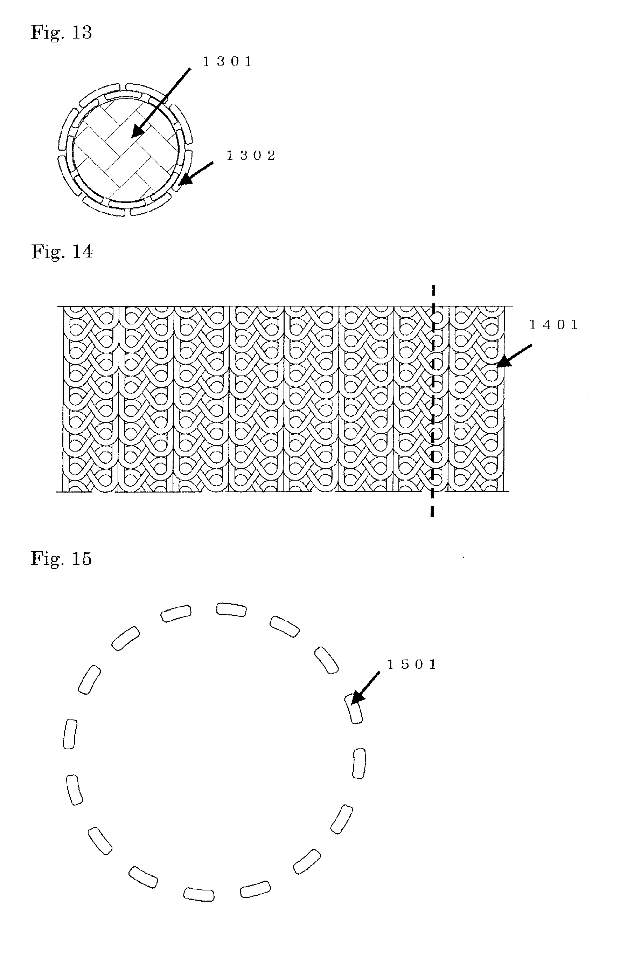

Figs. 13 through 16 show one embodiment of the present invention. For one embodiment of the present invention, the stent is an intraluminal stent in which nearly triangular shapes continuously form a cylindrical loop element, and adjacent struts overlap together in the radius direction in the folded first radius, and when a certain cross-section of the intraluminal stent is observed, struts are folded in two layers, outside and inside, in the radius direction. Furthermore, in the dilated second radius, an intraluminal stent does not overlap in the radius direction and is dilated nearly uniformly. Preferably, the intraluminal stent of the present embodiment is fabricated by stainless steel.


Examples


Referring now to drawings, examples of stent related to the present invention will be described as follows, but the present invention shall not be restricted to these.

One example of a method to place a stent is achieved by fixing the stent to a balloon portion at the head end of a catheter in the compressed state, allowing the stent to advance to a treated region in a patient's body lumen, dilating the balloon to dilate and place the stent, and then, decannulating the catheter. Consequently, two states are available for the stent, in the compressed state and in the dilated state. The stent is delivered in the compressed state, and placed in the patient's body lumen in the dilated state.

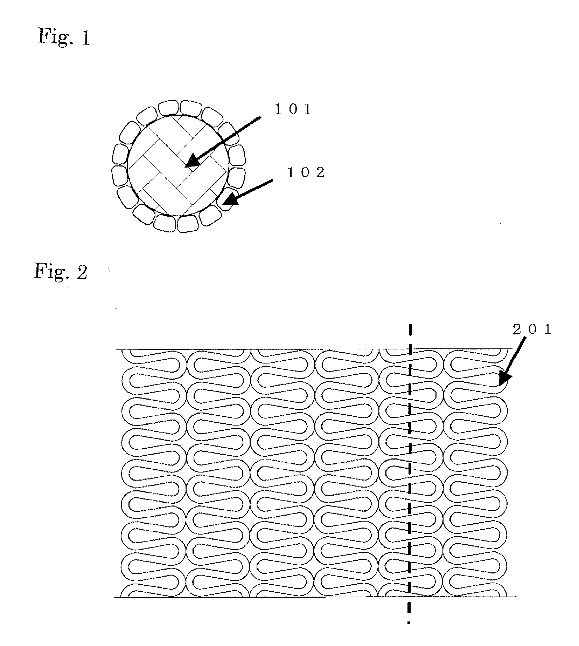

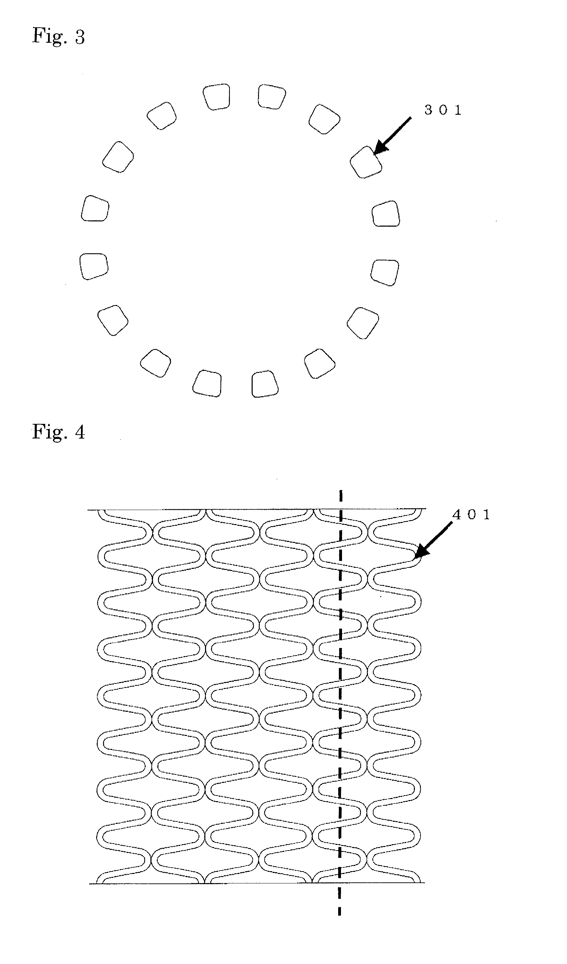

The following Figs. 1 through 4 indicate Comparison 1 as a typical example of a stent according to a conventional technology. Fig. 1 shows a cross-sectional view as seen from the stent axial direction when the stent of Comparison 1 is contracted. In this way in the conventional example, when a stent is fixed to a catheter or balloon, struts do not overlap in the radius direction and are fixed in a line generally in the circumferential direction. In addition, Fig. 2 shows a development view when the stent of Comparison 1 is contracted. Because in the conventional example, struts did not overlap in the radius direction in stent contraction, there existed no overlapping portion as seen in the development view. Fig. 3 is a cross-sectional view as seen from the axial direction of the stent after dilation of Comparison 1. Fig. 4 is a development view after stent dilation of Comparison 1.

In the conventional example, various examples with varying strut designs (geometrical shapes) are studied and put into market in addition to Comparison 1, but all of them do not have overlapping struts in the radius direction both in the compressed state and in the dilated state as shown in Comparison 1. The stent of Comparison 1 was fabricated by the use of metal (stainless steel) prescribed in JIS-G4303. The strut measures 100 $\mu$m in width and 100 $\mu$m in thickness, and the stent measures 13 mm in length.

Figs. 5 through 8 show Example 1 as an example of a stent related to the present invention. Fig. 5 is a cross-sectional view as viewed from the stent axial direction when the stent of Example 1 is contracted. In this way, in Example 1, struts are fixed with overlaps in the radius direction when the stent is fixed to a catheter or a balloon. Fig. 6 is a development view when the stent of Example 1 is contracted. In the development view, a portion with struts partially overlapping exists. Fig. 7 is a cross-sectional view as seen from the stent axial direction after dilation of Example 1. In addition, Fig. 8 is a development view after dilation of Example 1. After dilation, same as Comparison 1 , a conventional example, struts do not have any overlaps in the radius direction. The stent of Example 1 was fabricated by the use of metal (cobalt chromium alloy) prescribed in ISO5832-5. The strut measures 120 $\mu$m in width and 80 $\mu$m in thickness, and the stent measures 13 mm in length.

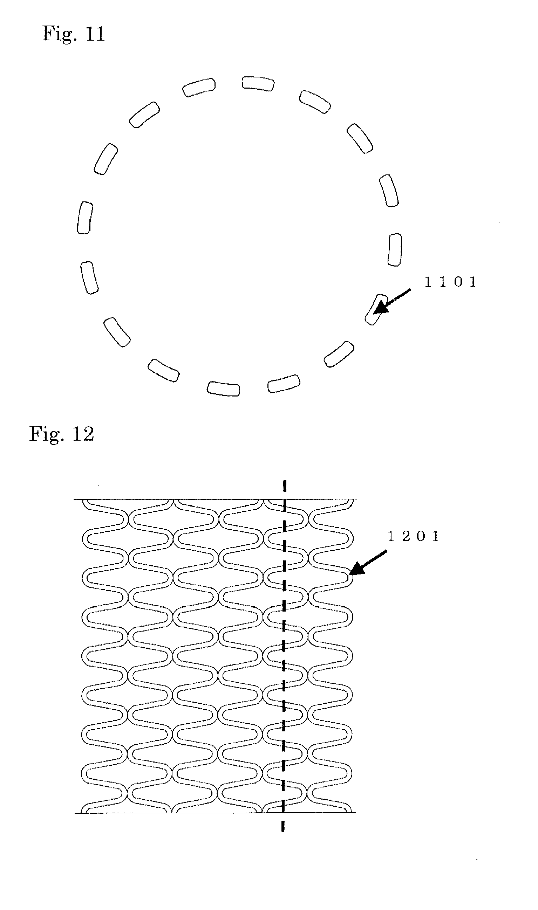

Figs. 9 through 12 show Example 2 as an example of a stent related to the present invention. Fig. 9 is a cross-sectional view as viewed from the stent axial direction when the stent of Example 2 is contracted. In this way, in Example 2, when the stent is fixed to a catheter or a balloon, struts are fixed with overlaps in the radius direction, and fixed partially in the two-layer state. Fig. 10 is a development view when the stent of Example 2 is contracted. In the development view, there exists a portion with partially overlapping struts. Fig. 11 is a cross-sectional view as seen from the stent axial direction after dilation of Example 2. In addition, Fig. 12 is a development view after dilation of Example 2. After dilation, same as Comparison 1, a conventional example, struts do not have any overlaps in the radius direction. The stent of Example 2 was fabricated by the use of metal (cobalt chromium alloy) prescribed in IS05832-6. The strut measures 140 $\mu$m in width and 70 $\mu$m in thickness, and the stent measures 13 mm in length.

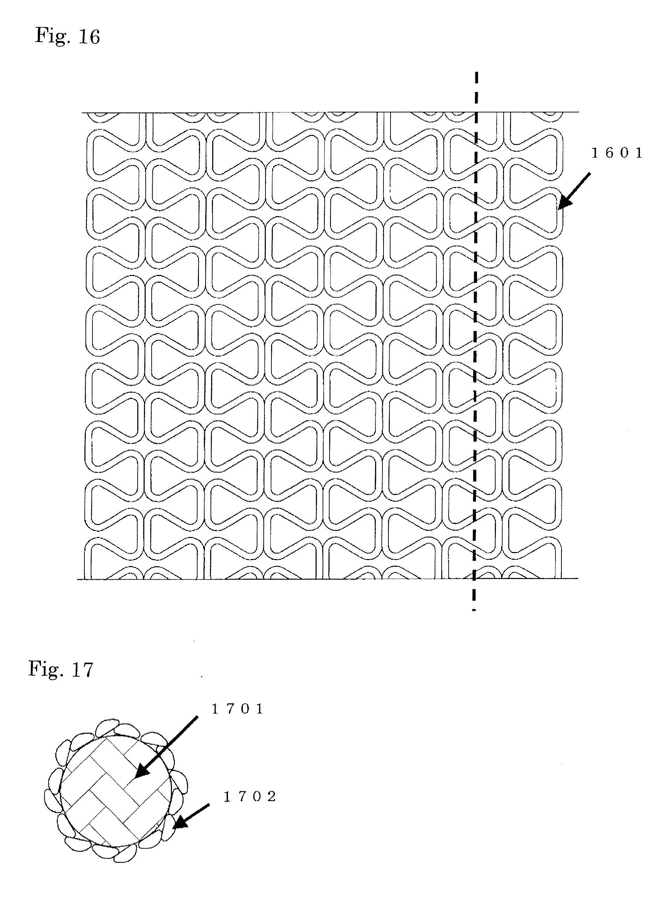

Figs. 13 through 16 show Example 3 as an example of a stent related to the present invention. Fig. 13 is a cross-sectional view as viewed from the stent axial direction when the stent of Example 3 is contracted. In this way, in Example 3, when the stent is fixed to a catheter or a balloon, struts are fixed with overlaps in the radius direction, and fixed partially in the two-layer state. Fig. 14 is a development view when the stent of Example 3 is contracted. Fig. 15 is a cross-sectional view as seen from the stent axial direction after dilation of Example 3. In addition, Fig. 16 is a development view after dilation of Example 3. After dilation, same as Comparison 1, a conventional example, struts do not have any overlaps in the radius direction. The stent of Example 3 was fabricated by the use of metal (cobalt chromium alloy) prescribed in ISO5832-7. The strut measures 200 $\mu$m in width and 50 $\mu$m in thickness, and the stent measures 13 mm in length.

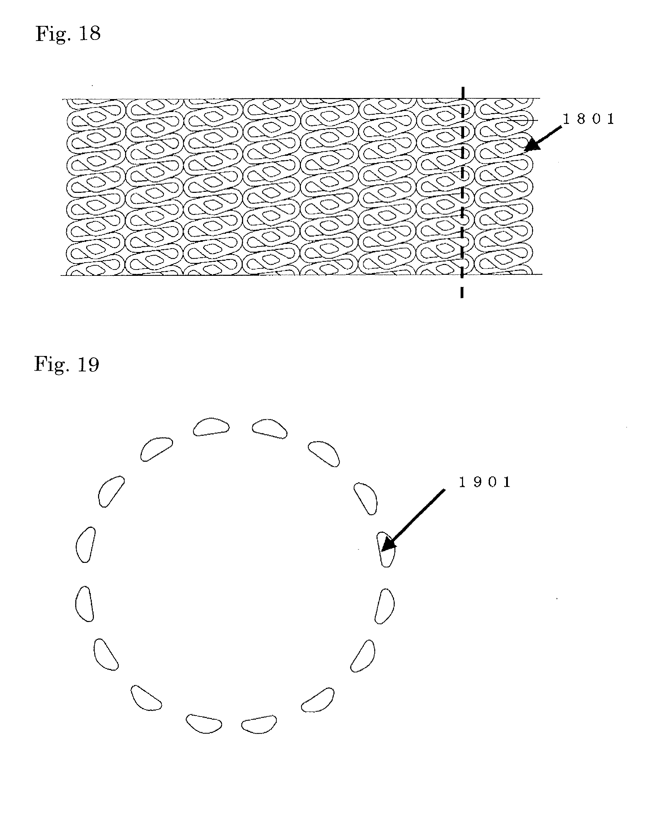

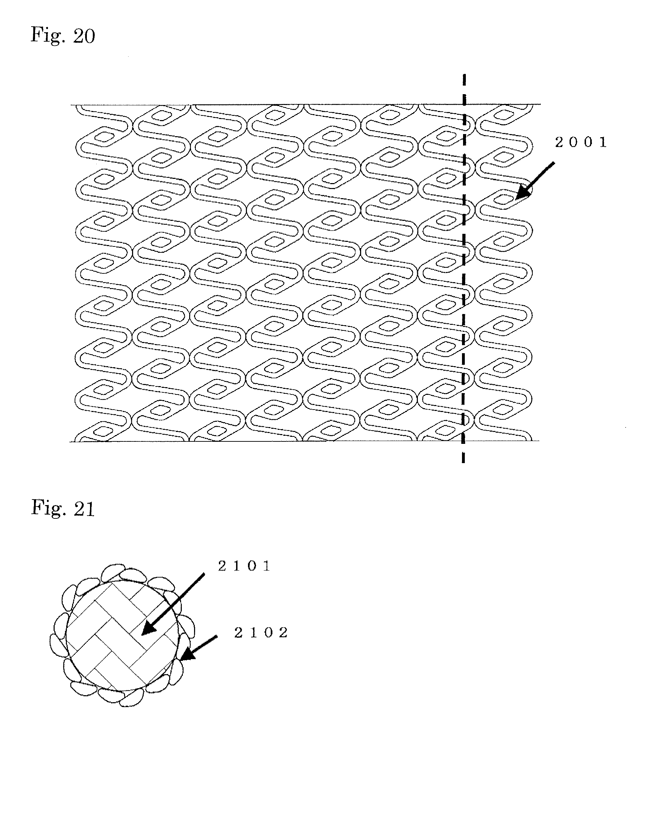

Figs. 17 through 20 show Example 4 as an example of a stent related to the present invention. Fig. 17 is a cross-sectional view as viewed from the stent axial direction when the stent of Example 4 is contracted. In this way, in Example 4, when the stent is fixed to a catheter or a balloon, struts are fixed with overlaps in the radius direction, and fixed partially in the two-layer state. Fig. 18 is a development view when the stent of Example 4 is contracted. Fig.19 is a cross-sectional view as seen from the stent axial direction after dilation of Example 4. In addition, Fig. 20 is a development view after dilation of Example 4. After dilation, same as Comparison 1, a conventional example, struts do not have any overlaps in the radius direction. The stent of Example 4 was fabricated by the use of metal (cobalt chromium alloy) prescribed in IS05832-7. The strut measures 250 $\mu$m in width and 40 $\mu$m in thickness, and the stent measures 13 mm in length.

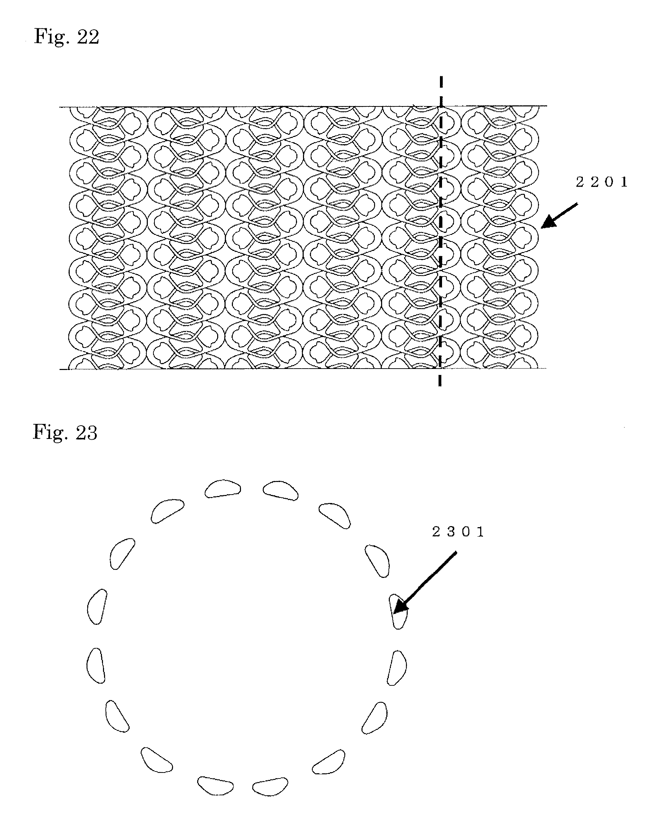

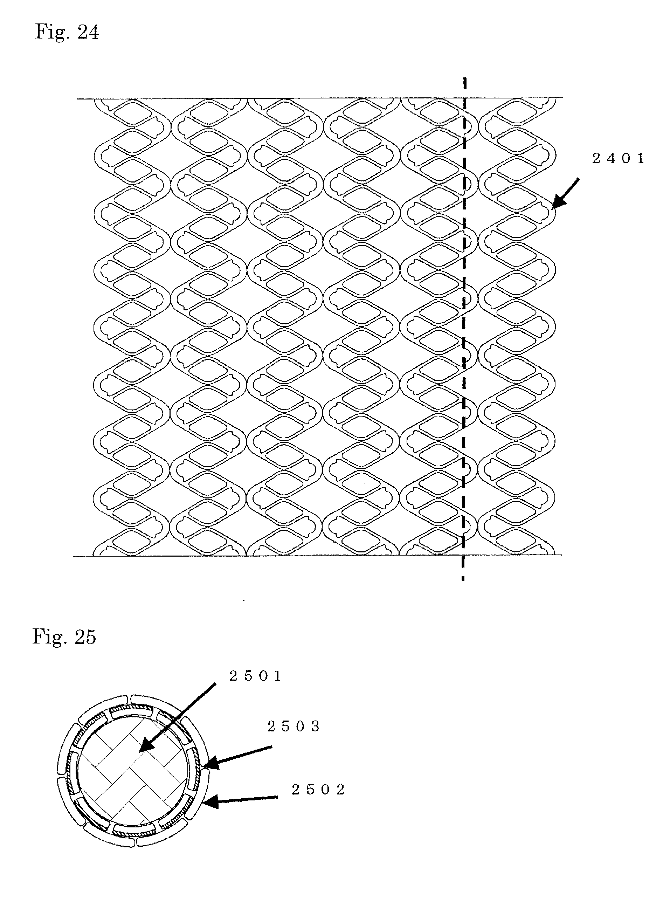

Figs. 21 through 24 show Example 5 as an example of a stent related to the present invention. Fig. 21 is a cross-sectional view as viewed from the stent axial direction when the stent of Example 5 is contracted. In this way, in Example 5, when the stent is fixed to a catheter or a balloon, struts are fixed with overlaps in the radius direction, and fixed partially in the two-layer state. Fig. 22 is a development view when the stent of Example 5 is contracted. Fig. 23 is a cross-sectional view as seen from the stent axial direction after dilation of Example 5. In addition, Fig. 24 is a development view after dilation of Example 5. After dilation, same as Comparison 1, a conventional example, struts do not have any overlaps in the radius direction. The stent of Example 5 was fabricated by the use of metal (cobalt chromium alloy) prescribed in ISO5832-7. The strut measures 300 $\mu$m in width and 33 $\mu$m in thickness, and the stent measures 13 mm in length.

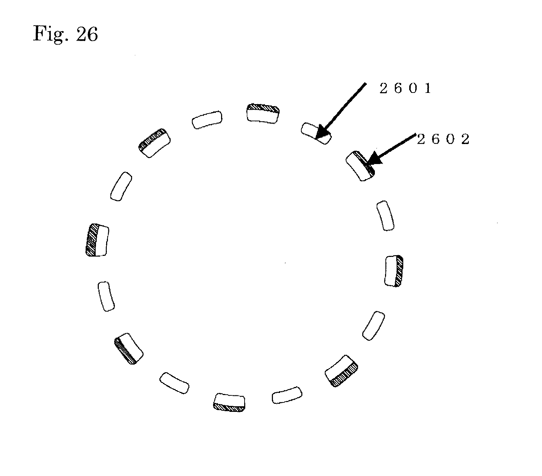

Figs. 25 and 26 show Example 6 as an example of a stent related to the present invention. For Example 6, biocompatibility polymer and a drug that suppresses occlusion were applied to the stent of Example 2. For a biocompatibility polymer, copolymer of polylactic-polyglycolic acid, which is a biodegradable polymer, too (available from SIGMA; lactic acid/glycolic acid = 85/15; weight-average molecular weight: 90,000-126,000), was used and tacrolimus (FK506) known as an immunosuppressant was used as a drug to suppress occlusion. Tacrolimus (FK506) is a compound of CAS No. 104987-11-3, and is disclosed, for example, in Japanese Patent Application Laid-Open Publication No. S61-148181. Tacrolimus(FK506)forms a complex together with FK506 binding protein (FKBP) in the cell, and is assumed to block the production of cytokines, namely IL-2 and INF-$\gamma$, which are primarily differentiation/proliferative factors from T-cells, and it is known that it can be used as a preventative drug or curative drug of immunologic rejection in association with organ transplantation or autoimmune disease. A polylactic-polyglycolic acid copolymer and tacrolimus were dissolved in chloroform, and solutions whose concentrations were 0.5 wt%, respectively, were prepared and were applied to the portions to be applied by brush to prepare the stents.

Fig. 25 is a cross-sectional view as viewed from the stent axial direction when the stent of Example 6 was contracted, and Fig. 26 is a cross-sectional view as viewed from the stent axial direction when the stent of Example 6 was dilated. In this way, such stents that the drug did not exist on the outer surface of the stent when the stent was in the first diameter but the drug existed on the outer surface of the stent when the stent was in the second diameter were enabled.

For Comparison 1 and Examples 1 through 6 as described above, the holding force between this balloon and the stent were compared and evaluated. The holding force referred to here is the force necessary to move the compressed and fixed stent from the balloon portion of the catheter. First, stents to be evaluated were all compressed and fixed to balloon catheters. Now, Rapid Exchange type Balloon Catheters (Medical Device Approval No. 21200BZZ00020000) commercially available from Kaneka Corporation were used for the balloon catheters. Evaluation was carried out in a 37° C water bath, a tensile tester was fixed to the stents by the use of grips, and the shaft portion of the balloon catheter was separately fixed. The tensile tester side was allowed to slide in the pulling direction by the use of an apparatus and the force necessary for the stent to be moved from the balloon portion was measured. Measurement of n=3 was conducted for each group of Comparison 1 and Examples 1 through 6, and the average values were shown in Table 1.

[Table 1]

| | |
|---|---|
| Example 1 | 5.29N |
| Example 2 | 5.20 N |
| Example 3 | 7.71 N |
| Example 4 | 6.58 N |
| Example 5 | 7.64 N |
| Example 6 | 6.46 N |
| Comparison 1 | 4.13 N |

The results indicated that all the Examples 1 through 6 of the present invention exhibited higher holding force than Comparison.

For Comparison 1 and Examples 1 through 6 discussed above, stent placement experiments using miniswine (Crown, female, 8 to 12 months old) were conducted and evaluated. Under anesthesia, a sheath (6Fr) was inserted in the right femoral artery of miniswine and the head end of a guiding catheter inserted from the sheath (6Fr) was allowed to engage with the left coronary ostium. After the stent was delivered to the left anterior descending coronary artery and left circumflex coronary artery via the guiding catheter, the stent was dilated and placed. After decannulating the guiding catheter and the sheath, the right femoral artery was ligated to stop bleeding. At the portion where the stent was placed, the stent was allowed to dwell in such a manner that about 1.25 was achieved for a ratio of the stent diameter to the blood vessel diameter. One each of stent was placed randomly in each blood vessel of the left anterior descending coronary artery and left circumflex coronary artery, as well as right coronary artery. From a day before the placement test to the day of necropsy, 330 mg of aspirin and 250 mg of Ticlopidine were administered by mixing in feed in a day. Twenty-eight days after placement, miniswine were put to sleep and their hearts were taken out. For each group of Comparison 1 and Examples 1 through 6 , n=3 of stents were placed and evaluated. In all the groups and all the stents, no problem occurred in stent placement manipulation and no problem such as stent occlusion occurred for 28 days of placement period. The coronary arteries to which stents were placed were removed from hearts and immersed and fixed in the 10% formalin neutral buffer solution. After resin-embedding, a segment of the center portion of each stent was prepared, were H. E. stained (hematoxylin-eosin stained), and examined by a magnifying glass. With the degree of damage of stent struts to the blood vessel used for an evaluation index, damage was scored for each strut, and the average of all struts was designated as the degree of damage of the stent. The damage score is zero when the stent does not come in contact with the elastic lamina in the blood vessel, score 1 when the strut comes in contact with the elastic lamina in the blood vessel but the inner elastic lamina is free from damage, score 2 when the strut penetrates the inner elastic lamina and comes in contact with the tunica media, score 3 when the strut comes in contact with the elastic lamina outside the blood vessel, and score 4 when the strut penetrates the outer elastic lamina and comes in contact with the tunica externa (the rating method was quoted from the method disclosed in Kornowsk et al., JACC. 1988; Vol 31, No. 1: 224-230). Table 2 shows the average of degree of damage of each group.

[Table 2]

| | |
|---|---|
| Example 1 | 1.7 |
| Example2 | 1.6 |
| Example3 | 0.7 |
| Example4 | 1.1 |
| Example5 | 0.6 |
| Example6 | 1.3 |
| Comparison1 | 2.2 |

The results indicate that all Examples 1 through 6 of the present invention provided lower degree of damage than Comparison.

Then, the blood vessel occlusion ratio of each stent group was compared. The lumen area (LA) of each stent cross section and the area within the internal elastic lamina (IELA) were measured. Using the lumen area (LA) and the area within the internal elastic lamina (IELA), the blood vessel occlusion ratio was calculated in accordance with the following formula.

$$\text{Formula: Blood vessel occlusion ratio (\%)} = (1-(LA/IELA)) \times 100$$

Table 3 shows the average of blood vessel occlusion ratio of each group.

[Table 3]

| | |
|---|---|
| Example 1 | 45.9% |
| Example2 | 47.5% |
| Example3 | 36.5% |
| Example4 | 38.9% |
| Example5 | 35.6% |
| Example6 | 21.6% |
| Comparison1 | 57.2% |

The results indicate that all Examples 1 through 6 of the present invention provided lower blood vessel occlusion ratio than Comparison. In addition, with Example 6 in which Tacrolimus (FK506) was applied as a drug to suppress occlusion, marvelous drop of the occlusion ratio was observed.

**Claims**

1. An intraluminal stent which is able to be dilated in the radius direction from a compressed first diameter to a dilated second diameter, and in the state of the first diameter, struts constituting the intraluminal stent overlap together in the radius direction at least in one part.

2. The intraluminal stent according to claim 1, wherein the struts constituting the intraluminal stent do not overlap together in the radius direction in the state of the second diameter.

3. The intraluminal stent according to claim 2, wherein an area of the portion with overlapping struts is larger than an area of the portion with no overlapping struts in the state of the first diameter.

4. The intraluminal stent according to claim 1 , wherein struts constituting the intraluminal stent are configured in nearly wave-like shapes.

5. The intraluminal stent according to claim 1 , wherein the intraluminal stent includes multiple cylindrical loop elements which can be independently dilated in the radius direction and the cylindrical loop elements are formed continuously nearly in the axial direction.

6. The intraluminal stent according to claim 1, wherein the stent is formed by materials selected from stainless steel, nickel alloy, cobalt chromium alloy, and combinations of these .

7. The intraluminal stent according to claim 1, wherein a drug that suppresses occlusion is fixed.

8. The intraluminal stent according to claim 7, wherein the drug is fixed by biocompatible polymer.

9. The intraluminal stent according to 7, wherein the drug is fixed by biodegradable polymer.

10. The intraluminal stent according to claim 7, wherein the drug does not exist on the outer surface of the stent in the state of the first diameter but the drug exists on the outer surface of the stent in the state of the second diameter.

Fig. 1

1 0 1

1 0 2

Fig. 2

2 0 1

Fig. 3

3 0 1

Fig. 4

4 0 1

Fig. 5

5 0 1

5 0 2

Fig. 6

6 0 1

Fig. 7

7 0 1

Fig. 8

801

Fig. 9

901

902

Fig. 10

1001

Fig. 11

1 1 0 1

Fig. 12

1 2 0 1

Fig. 13

Fig. 14

Fig. 15

Fig. 16

1 6 0 1

Fig. 17

1 7 0 1

1 7 0 2

Fig. 18

1 8 0 1

Fig. 19

1 9 0 1

Fig. 20

2 0 0 1

Fig. 21

2 1 0 1

2 1 0 2

Fig. 22

2 2 0 1

Fig. 23

2 3 0 1

Fig. 24

2 4 0 1

Fig. 25

2 5 0 1

2 5 0 3

2 5 0 2

Fig. 26

2 6 0 1

2 6 0 2

## INTERNATIONAL SEARCH REPORT

International application No.

PCT/JP2005/013754

A. CLASSIFICATION OF SUBJECT MATTER
**A61F2/84** (2006.01), **A61M29/02** (2006.01)

According to International Patent Classification (IPC) or to both national classification and IPC

B. FIELDS SEARCHED

Minimum documentation searched (classification system followed by classification symbols)
**A61F2/84** (2006.01), **A61M29/02** (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 2003/061528 A1 (SCIMED LIFE SYSTEMS, INC.),<br>31 July, 2003 (31.07.03),<br>Claims; all drawings<br>JP 2005-515022 A          & US 2003/0139799 A1<br>& EP 1469792 A1 | 1-9<br>10 |
| X<br>A | JP 2772665 B2 (Nippon Zeon Co., Ltd.),<br>02 July, 1998 (02.07.98),<br>Claims; Fig. 2<br>(Family: none) | 1-9<br>10 |
| X<br>A | JP 3-277377 A (Olympus Optical Co., Ltd.),<br>09 December, 1991 (09.12.91),<br>Claims; Figs. 14 to 15<br>(Family: none) | 1-9<br>10 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>01 November, 2005 (01.11.05) | Date of mailing of the international search report<br>15 November, 2005 (15.11.05) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2005/013754 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | JP 2004-515307 A  (Riva Medikaru Inc.),<br>27 May, 2004 (27.05.04),<br>Claims; all drawings<br>& WO 2002/047582 A2      & US 2003/0199969 A1<br>& EP 1341481 A2 | 1,3-9 |
| A | WO 2003/090810 A1  (MEDTRONIC AVE, INC.),<br>06 November, 2003 (06.11.03),<br>Claims; all drawings<br>& JP 2005-523747 A      & US 2003/0204245 A1<br>& EP 1499370 A1 | 10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H2174859 A
- JP H6181993 A
- JP H10503676 A
- JP H11319112 A
- JP H11501551 A
- JP 2001224696 A
- JP 2001501494 A
- JP 2001501493 A

- JP 2002530146 A
- JP H5502179 B
- JP H69390 A
- JP H9503488 B
- WO 02065947 A
- EP 1254674 A
- JP 61148181 A

**Non-patent literature cited in the description**

- **PACHE J. et al.** *J Am Coll Cardiol,* 16 April 2003, vol. 41 (8), 1289-92

- **KORNOWSK et al.** *JACC,* 1988, vol. 31 (1), 224-230